# EUROPÄISCHE PATENTSCHRIFT

(11) **EP 0 682 919 B1**
(45) Veröffentlichungstag und Bekanntmachung des Hinweises auf die Patenterteilung: **30.01.2002**
(21) Anmeldenummer: 95106887.3
(22) Anmeldetag: 06.05.1995
(51) Int. Cl.: A61B 19/00

(54) **Verfahren zum Korrelieren verschiedener Koordinatensysteme in der rechnergestützten, stereotaktischen Chirurgie**
Method for correlating several coordinate systems in computer aided stereotactic surgery
Méthode pour la corrélation de plusieurs systèmes de coordinates dans la chirurgie stéréotactique assistée par ordinateur

(30) Priorität: 21.05.1994 DE 4417944
(43) Veröffentlichungstag der Anmeldung: 22.11.1995
(73) Patentinhaber: Carl Zeiss, 89518 Heidenheim (Brenz) (DE)
(72) Erfinder: Luber, Joachim, D-73457 Essingen-Forst (DE); Mackevics, Arvids, D-73432 Aalen-Unterkochen (DE)

(56) Entgegenhaltungen:
- DE-A- 4 134 481
- US-A- 5 099 846

## Beschreibung

Die vorliegende Erfindung betrifft ein Verfahren zum Korrelieren verschiedener Koordinatensysteme in der rechnergestützten, stereotaktischen Chirurgie nach dem Oberbegriff von Anspruch 1.

Ein derartiges Verfahren ist in der DE 41 34 481 A1 summarisch angesprochen, wobei die DE 41 34 481 A1 keinen Hinweis darauf gibt, wie ein derartiges Verfahren tatsächlich durchgeführt werden könnte.

In der rechnergestützten, stereotaktischen Chirurgie ist es erforderlich, verschiedene Koordinatensysteme miteinander zu korrelieren, wenn präoperativ generierte Diagnosedaten, die z.B. mittels einer NMR-Tomographie-Anordnung erzeugt wurden, zur Orientierungs-Optimierung während einer Operation herangezogen werden sollen. Dies ist beispielsweise dann nötig, wenn derartige bildmäßige Informationen einem aktuell gewonnenen Bild des Operationsfeldes überlagert werden sollen. Das aktuelle Bild des Operationsfeldes kann dabei das über ein Operationsmikroskop betrachtete Sehfeld sein oder aber auch ein Monitorbild, welches über Video-Kameras erzeugt wurde.

Üblicherweise erfolgt das erforderliche Korrelieren über eine Referenzierungs-Prozedur, bei der sogenannte Referenzierungs-Marker am Patienten vermessen werden, deren koordinatenmäßige Positionen relativ zum Patienten-Koordinatensystem und zum Diagnosedaten-Koordinatensystem bekannt sind. Ein derartiges Vermessen von Referenzierungs-Markern kann z.B. mit einem Operationsmikroskop erfolgen, wie es aus der DE 41 34 481 der Anmelderin bekannt ist.

Alternativ kann eine Vermessung der Referenzierungs-Marker auch mit Hilfe von sog. Laser-Pointern erfolgen, die am Operationsmikroskop angeordnet werden.
Ferner ist aus der US 5,279,309 bekannt, derartige Referenzierungs-Marker als Mini-Sender auszuführen und am Patienten angeordnet über entsprechende Detektoren zu lokalisieren.

Nachteilig ist jedoch bei diesen bekannten Verfahren, die ein Referenzieren mittels Referenzierungs-Markern vorsehen, daß diese Verfahren relativ zeitaufwendig sind und nach einer eventuellen Positionsänderung des Patienten wiederholt werden müssen.

Ferner ist es jeweils erforderlich, die vorzugsweise bildgebenden Diagnoseverfahren mit diesen Referenzierungs-Markern vorzunehmen. Dies wiederum stellt eine Belastung für den Patienten dar, wenn etwa diese Referenzierungs-Marker implantiert werden müssen, wie es z.B. in der US 5,178,164 beschrieben wird.

Es ist daher die Aufgabe der vorliegenden Erfindung, ein Verfahren explizit anzugeben, das es ermöglicht, die präoperativ generierten Diagnosedaten mit den aktuellen topographischen Patientendaten unter Verwendung eines Operationsmikroskopes zu korrelieren, ohne daß die koordinatenmäßige Vermessung bzw. Lokalisierung separater Referenzierungs-Marker erforderlich ist. Insbesondere sollen hierbei die stereotaktischen Einsatzmöglichkeiten eines Operationsmikroskopes verbessert werden.

Diese Aufgabe wird gelöst durch ein Verfahren mit den Merkmalen des Anspruches 1.
Vorteilhafte Ausgestaltungen des erfindungsgemäßen Verfahrens finden sich in den Unteransprüchen.

Es werden nunmehr keine separaten Referenzierungs-Marker verwendet, um in zeitaufwendigen Referenzierungs-Prozeduren das Korrelieren der verschiedenen Koordinatensysteme vorzunehmen. Vielmehr dient das an einem geeigneten Trägersystem angeordnete Operationsmikroskop unmittelbar dazu, zumindest Topographie-Teilinformationen des Patienten zu erfassen. Die mittels des Operationsmikroskopes gewonnenen und anschließend digitalisierten Topographie-Teilinformationen werden anschließend über bekannte Algorithmen mit den präoperativ erstellten Diagnose-Datensätzen korreliert.

Zum Gewinnen der präoperativen Diagnose-Datensätze können dabei verschiedenste bildgebende Verfahren in Kombination mit dem erfindungsgemäßen Verfahren zum Einsatz kommen. Stellvertretend seien dabei die Kernspin-Tomographie (NMR), die Positronen-Emissions-Tomographie (PET), die Magnet-Enzephalographie (MEG) sowie die Single-Photonen-Emissions-Computer-Tomographie (SPECT) als moderne Diagnoseverfahren erwähnt, die sowohl einzeln als auch in verschiedensten Kombinationen zum Einsatz kommen können.

Das Gewinnen der Topographie-Teilinformationen mit Hilfe des Operationsmikroskopes kann auf verschiedene Art und Weise erfolgen. Je nach gewünschtem Aufwand und spezifischen Anforderungen kann eine geeignete Ausführungsform des erfindungsgemäßen Verfahrens gewählt werden.
Entsprechend dem jeweiligen Verfahren ist das eingesetzte Operationsmikroskop auszulegen.

Es entfallen numehr die zeitaufwendigen Referenzierungs-Prozeduren mit separaten Referenzierungs-Markern und die daraus resultierenden aufwendigen Bedienungsabläufe inclusive der entsprechenden Patientenbelastung, wenn beispieleswiese ein Implantieren derartiger Referenzierungs-Marker erforderlich ist.

Ferner ist durch das nahezu automatisierte Erfassen von Topographie-Teilinformationen mit Hilfe des Operationsmikroskopes auch bei Veränderung der aktuellen Patientenposition oder Körperteilen des Patienten ein rasches erneutes Korrelieren der verschiedenen Koordinatensysteme möglich.

Weitere Vorteile und Einzelheiten des erfindungsgemäßen Verfahrens ergeben sich aus der nachfolgenden Beschreibung eines Ausführungsbeispiels anhand der beiliegenden Figuren.

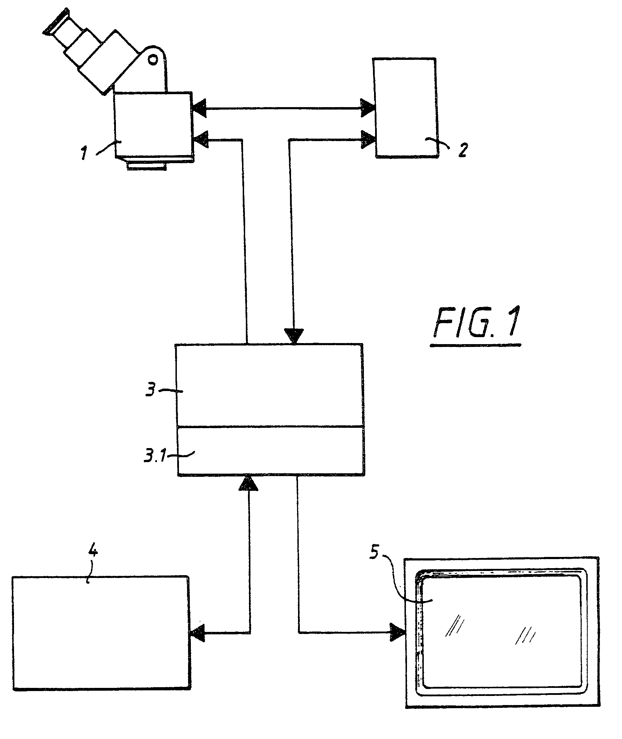

Dabei zeigt
- Figur 1: ein Blockschaltbild mit den wichtigsten erforderlichen Komponenten für die Durchführung des erfindungsgemäßen Verfahrens;
- Figur 2: ein Flußdiagramm eines Ausführungsbeispieles des erfindungsgemäßen Verfahrens;

Zur Durchführung des erfindungsgemäßen Verfahrens ist ein Operationsmikroskop (1) vorgesehen, das an einem geeigneten - lediglich schematisiert angedeuteten - Trägersystem (2) angeordnet wird, das im dargestellten Ausführungsbeispiel motorisch betrieben wird.

Mit Hilfe des Trägersystemes (2) ist das definierte, koordinatenmäßig-bestimmte Positionieren des Operationsmikroskopes (1) in bis zu sechs räumlichen Freiheitsgraden möglich. Üblich sind hierbei drei Rotationssowie drei Translationsfreiheitsgrade für die räumlichen Positionierungsmöglichkeiten des Operationsmikroskopes.

Ein geeignetes, mehrgelenkiges Trägersystem (2), das motorisch betrieben wird, ist etwa in der DE 42 02 922 der Anmelderin dargestellt. Dieses umfaßt u.a. den jeweiligen Gelenken zugeordnete Antriebe sowie Encoder, so daß einer zentralen Steuereinheit (3) des Gesamtsystemes laufend ein Ermitteln der koordinatenmäßigen Position des Operationsmikroskopes (1) zusammen mit den bekannten Geometrie-Informationen des Trägersystemes (2) und des Operationsmikroskopes (1) möglich ist.

Alternativ zum bekannten Trägersystem aus der DE 42 02 922 ist auch der Einsatz einfacher aufgebauter Trägersysteme möglich, sofern diese ein definiertes räumliches Positionieren des daran angeordneten Operationsmikroskopes sowie das Erfassen der jeweiligen koordinatenmäßigen Position ermöglichen. Hierbei ist etwa denkbar, in bestimmten Ausführungsformen des erfindungsgemäßen Verfahrens auf die Antriebe der einzelnen Gelenke zu verzichten und das Trägersystem manuell zu positonieren oder aber eine alternative Positionserfassung vorzusehen.

Das verwendete Operationsmikroskop (1) weist einen, im wesentlichen bekannten, optischen Aufbau auf und kann u.a. eine Einspiegelungs-Vorrichtung umfassen, über die eine Einspiegelung anderweitig generierter Bilder oder graphischer Darstellungen über bekannte Strahlteiler-Vorrichtungen in mindestens einem der stereoskopischen Beobachtungs-Strahlengänge möglich ist.

Desweiteren umfaßt das Operationsmikroskop (1) ein Positionserkennungssystem auf optischer Basis, mit dem ein aktives Vermessen der gerade betrachteten Sehfeldebene mit hoher Präzision möglich ist. Beispielsweise kann hierzu ein Laser-Triangulations-Meßsystem in die Operationsmikroskop-Optik integriert werden, mit dem ein Punkt in der Sehfeldebene koordinatenmäßig präzise erfaßt werden kann.

Hinsichtlich eines bevorzugt einzusetzenden, geeigneten Operationsmikroskopes sei ausdrücklich auf das in der DE 41 34 481 der Anmelderin beschriebene Operationsmikroskop verwiesen.

Alternativ zum Positionserkennungssystem auf optischer Basis sind ggf. auch andere, vorzugsweise ebenfalls kontaktlos arbeitenden Positionserkennungssysteme erfindungsgemäß einsetzbar. Möglich ist hierbei etwa der Einsatz von Ultraschall-3D-Digitizern etc..

Desweiteren ist eine - bereits erwähnte - zentrale Steuereinheit (3) in Form eines Steuer-Rechners vorgesehen. Die Steuereinheit (3) übernimmt sowohl die komplette Steuerung und Positionserfassung des Trägersystemes als auch die Verarbeitung der Bildinformationen aus den präoperativen Diagnose-Datensätzen, die in einer geeigneten Bild-Datenbank (4) digitalisiert abgelegt wurden.

Die zentrale Steuereinheit (3) umfaßt desweiteren eine Eingabeschnittstelle (3.1), z.B. in Form einer Tastatur, über die der Benutzer beispielsweise die Operationsplanung vornimmt etc..

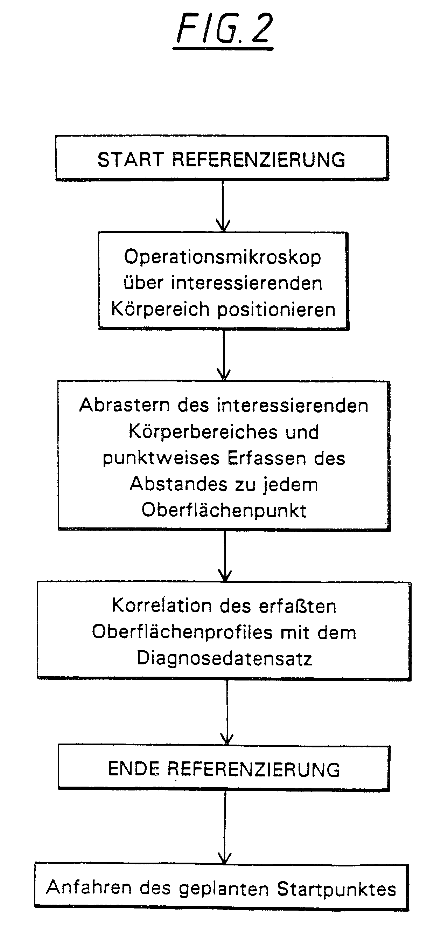

Ein Ausführungsbeispiel des erfindungsgemäßen Verfahrens wird nunmehr anhand des Flußdiagrammes in Figur 2 erläutert, in dem wichtige Verfahrensschritte aufgelistet sind.
Das an einem nunmehr motorisch-betriebenen Trägersystem angeordnete Operationsmikroskop wird in einer ersten Ausführungsform des erfindungsgemäßen Verfahrens zunächst definiert über den interessierenden und zu korrelierenden Körperbereich am Patienten verfahren. Hierzu erfolgt zunächst die Selektion dieses Körperbereiches und das entsprechende Positionieren des Operationsmikroskopes über diesem Körperbereich mit Hilfe des motorischen Trägersystemes.

Die Sehfeldebene des Operationsmikroskopes wird anschließend in einer Scan-Bewegung über den interessierenden Körperbereich gerastert. Die Rasterbewegung kann hierbei durch manuellgesteuertes Verfahren des am motorischen Trägersystem angeordneten Operationsmikroskopes durch den Benutzer erfolgen. Alternativ ist es aber auch möglich, daß die zentrale Steuereinheit ein definiertes Verfahren über diesen Bereich mit bestimmten vorzugebenden Verfahr-Parametern übernimmt. Zu diesen Verfahrparametern zählen etwa die Vorgabe des definierten Scan-Bereiches sowie die Scan-Geschwindigkeit usw.. Während der Scan-Bewegung über den interessierenden Körperbereich, der mit dem präoperativen Diagnose-Datensatz räumlich zu korrelieren ist, wird mit Hilfe des Positionserkennungssystems des Operationsmikroskopes punktweise laufend der Abstand der Objektoberfläche zum Operationsmikroskop bestimmt, dessen Raumkoordinaten der zentralen Steuereinheit aufgrund der Encoder-Informationen des Trägersystemes stets bekannt sind. Auf diese Art und Weise ist es somit möglich, Topographie-Teilinformationen des interessierenden Köperbereiches am Patienten zu erfassen und anschließend digitalisiert abzulegen. Die derart gewonnenen Topographie-Informationen bestehen in diesem Ausführungsbeispiel demzufolge aus einem dreidimensionalen Oberflächen-Profil, wie es aus den unterschiedlichen Entfernungen zwischen Objektoberfläche und Operationsmikroskop über einen gewissen Flächenbereich generiert wurde. Die so mit Hilfe des Operationsmikroskopes gewonnenen Topographie-Teilinformationen im betrachteten bzw. interessierenden Objektbereich werden anschließend mit den Topographie-Informationen aus dem präoperativen Diagnose-Datensatz, der ebenfalls dreidimensionale Objektinformationen liefert, korreliert. Zur Korrelation des erfaßten 3D-Profiles mit dem präoperativ generierten Diagnose-Datensatz dienen etwa bekannte Korrelationsalgorithmen, wie sie z.B. auch aus der Photogrammetrie bekannt sind.

Diese Ausführungsform des erfindungsgemäßen Verfahrens benötigt weder separate Referenzierungs-Marker am Patienten, noch zusätzliche Vorrichtungen am eingesetzten Operationsmikroskop, wenn z.B. das Operatiosmikroskop aus der oben erwähnten DE 41 34 481 verwendet wird. Als einzige Anforderung an das eingesetzte Operationsmikroskop ist festzuhalten, daß damit eine Entfernungsmessung zu einem Punkt im Sehfeld und eine entsprechende Signal-Übergabe an die zentrale Steuereinheit realisierbar sein muß.

Möglich ist in dieser Ausführungsform des erfindungsgemäßen Verfahrens auch der Einsatz alternativer Operationsmikroskope, die z.B. mit einem aktiven Autofokus-System ausgestattet sind und damit die erforderliche punktweise Entfernungsmessung bewerkstelligen etc.

Nach der erfolgten Korrelation der erfaßten Patienten-Topographie mit den präoperativen Diagnosedaten ist die Referenzieungs-Prozedur beendet und es wird der geplante Startpunkt des Operationsmikroskopes für die anschließende Operation angefahren.

Ein Gewinnen von Topographie-Teilinformationen über die zuletzt beschriebene Ausführungsform des erfindungsgemäßen Verfahrens kann in vorteilhafter Weise intraoperativ eingesetzt werden, wenn z.B. bei Gehirnoperationen Gehirnverschiebungen resultieren und dann ein erneutes Referenzieren des betrachteten Sehfeldes mit den präoperativen Diagnosedatensätzen erforderlich ist.

## Patentansprüche

1. Verfahren zum Korrelieren verschiedener Koordinatensysteme in der rechnergesfützten, stereotaktischen Chirurgie ohne die Verwendung zusätzlicher Referenzierungs-Marker, wobei über ein an einem Trägersystem (2) angeordnetes Operationsmikroskop (1) mindestens Topographie-Teilinformationen gewonnen werden und die Topographie-Teilinformationen über einen Korrelationsalgorithmus mit präoperativ generierten Diagnosedaten räumlich korreliert werden,
**dadurch gekennzeichnet, daß** das Operationsmikroskop (1) über einen bestimmten Flächenbereich des beobachteten Objektes verfahren wird und dabei laufend punktweise die Abstände zu Oberflächenpunkten im interessierenden Körperbereich gemessen werden, um so ein dreidimensionales Oberflächenprofil dieses Körperbereiches zu generieren, wobei diese Informationen als Topographie-Teilinformationen zum anschließenden Korrelations-Algorithmus herangezogen werden.

2. Verfahren nach Anspruch 1, **dadurch gekennzeichnet, daß** das Operationsmikroskop (1) manuell vom Benutzer über dem interessierenden Körperbereich positioniert wird.

3. Verfahren nach Anspruch 1, **dadurch gekennzeichnet, daß** ein von einer zentralen Steuereinheit (3) automatisiertes Positionieren des Operationsmikroskopes (1) mit definierten, wählbaren Verfahrparameteren erfolgt, wobei das Operationsmikroskop (1) an einem motorisch betriebenen Trägersystem angeordnet ist.

## Claims

1. Method for the correlation of different coordinate systems in computer-assisted stereotactic surgery without the use of additional referencing markers, an operation microscope (1) arranged on a support system (2) being used to obtain at least partial topographical information and the partial topographical information being spatially correlated with preoperatively generated diagnosis data by a correlation algorithm, **characterized in that** the operation microscope (1) is moved over a specific area of the object being observed, during which the distances from surface points in the body region of interest are continuously measured pointwise, in order thereby to generate a three-dimensional surface profile of this body region, this information being employed as partial topographical information for the subsequent correlation algorithm.

2. Method according to Claim 1, **characterized in that** the operation microscope (1) is positioned manually by the user over the body region of interest.

3. Method according to Claim 1, **characterized in that** the operation microscope (1) is positioned automatically, with defined selectable method parameters, by a central control unit (3), the operation microscope (1) being arranged on a motorized support system.

## Revendications

1. Méthode pour la corrélation de plusieurs systèmes de coordonnée dans la chirurgie stéréotactique assistée par ordinateur sans l'utilisation de marqueur de référence supplémentaire, des informations topographiques partielles étant au moins obtenues par le biais d'un microscope d'opération (1) disposé sur un système porteur (2) et les informations topographiques partielles étant corrélées dans l'espace par le biais d'un algorithme de corrélation avec des données de diagnostic générées avant l'opération, **caractérisée en ce que** le microscope d'opération (1) est déplacé au-dessus d'une zone donnée de la surface de l'objet observé et que les distances par rapport aux points de la surface dans la zone concernée du corps sont ici mesurées continuellement afin de générer ainsi un profil tridimensionnel de la surface de l'objet, ces informations étant ensuite utilisées comme informations topographiques partielles pour l'algorithme de corrélation qui suit.

2. Méthode selon la revendication 1, **caractérisée en ce que** le microscope d'opération (1) est positionné manuellement par l'utilisateur au-dessus de la zone concernée du corps.

3. Méthode selon la revendication 1, **caractérisée en ce que** le positionnement du microscope d'opération (1) est réalisé automatiquement par un module de commande central (3) avec des paramètres de déplacement définis sélectionnables, le microscope d'opération (1) étant disposé sur un système porteur à entraînement motorisé.
